# EUROPEAN PATENT APPLICATION

(11) **EP 4 840 494 A1**
(43) Date of publication of application: **18.11.2026**
(21) Application number: 26175494.9
(22) Date of filing: 29.04.2026
(51) Int. Cl.: A61M 16/01, A61M 16/08

(54) **CALIBRATION APPARATUS AND CALIBRATION METHOD FOR DRIVE GAS CIRCUIT, AND MEDICAL DEVICE**

(30) Priority: 16.05.2025 CN 202510641242
(71) Applicant: GE Precision Healthcare LLC, Waukesha, WI 53188 (US)
(72) Inventor: FANG, Hui, Waukesha, 53188 (US); HE, Xiaolei, Waukesha, 53188 (US); LIU, Yiqiang, Waukesha, 53188 (US)
(74) Representative: Kilburn & Strode LLP

(57) **Abstract**

Embodiments of the present application provide a calibration apparatus and a calibration method for a drive gas circuit, and a medical device. The calibration apparatus includes: a first selector valve, the first selector valve switching communication between different passages; and a control unit, the control unit calibrating different components in the drive gas circuit by controlling switching of the first selector valve. Thus, the drive gas circuit can be calibrated without manual preparation, thereby improving the calibration accuracy and efficiency.

## Description

### TECHNICAL FIELD

Embodiments of the present application relate to the technical field of medical devices, and in particular, to a calibration apparatus and a calibration method for a drive gas circuit, and a medical device.

### BACKGROUND

In a scenario where a medical device is used to perform operations such as gas delivery on a patient, the medical device needs to be calibrated first, for example, the appearance, buttons, and drive gas circuit of the medical device need to be calibrated.

By way of example, components such as a drive gas pressure sensor, a flow control valve (also referred to as an inspiratory valve), and a flow restrictor need to be calibrated during calibration of the drive gas circuit of the medical device. However, preparations are necessary before calibrating these components. For example, prior to calibrating the drive gas pressure sensor, it is necessary to connect a three-way connector to the drive gas pressure sensor, connect a pressure test device to the three-way connector, and turn a switch to a ventilation position; prior to calibrating the flow control valve, it is necessary to connect an engine branch pressure tube to a pressure sensing connector of a control box, remove a bellows housing and a bellows, remount the bellows housing, disconnect a patient circuit, and turn the switch to the ventilation position; and prior to calibrating the flow restrictor, it is necessary to remove the bellows housing, the bellows, and edge components, and plug a drive gas port.

It should be noted that the above introduction of the background is only for the convenience of clearly and completely describing the technical solutions of the present application, and for the convenience of understanding for those skilled in the art.

### SUMMARY OF THE INVENTION

The inventor of the present application found that in the prior art, when a drive gas circuit in a medical device is calibrated, the calibration of different components in the drive gas circuit requires different preparations to be carried out in advance, such as disassembly and/or assembly operations of parts. This results in low calibration efficiency and a complicated calibration process. In addition, gas leakage risks may arise from the disassembly and/or assembly operations during preparation, which further leads to relatively low calibration accuracy.

To solve at least one of the above technical problems or other similar technical problems, embodiments of the present application provide a calibration apparatus and a calibration method for a drive gas circuit, and a medical device. The apparatus can calibrate different components in the drive gas circuit by switching communication between passages. Therefore, manual preparation is not needed, thereby simplifying the calibration process and improving the calibration efficiency and accuracy.

According to one aspect of the embodiments of the present application, a calibration apparatus for a drive gas circuit is provided. The calibration apparatus includes:
a first selector valve, where the first selector valve switches communication between different passages; and
a control unit, where the control unit calibrates different components in the drive gas circuit by controlling switching of the first selector valve.

According to another aspect of the embodiments of the present application, a calibration method for a drive gas circuit is further provided. The calibration method includes:
switching communication between different passages by a first selector valve; and
calibrating different components in the drive gas circuit by controlling switching of the first selector valve.

According to another aspect of the embodiments of the present application, a medical device is further provided, including a drive gas circuit, where the medical device further includes the calibration apparatus for the drive gas circuit as described above.

One of the beneficial effects of the embodiments of the present application is that: The calibration apparatus for the drive gas circuit calibrates the different components in the drive gas circuit according to the switching of communication between the different passages by the first selector valve. Thus, manual preparation is not needed before calibrating the various component in the drive gas circuit, thereby simplifying the calibration process and improving the calibration efficiency and accuracy.

With reference to the following description and drawings, specific implementations of the embodiments of the present application are disclosed in detail, and the way in which the principles of the embodiments of the present application can be employed are illustrated. It should be understood that the implementations of the present application are not limited in scope thereby. Within the scope of the spirit and clauses of the appended claims, the implementations of the present application comprise many changes, modifications, and equivalents.

### BRIEF DESCRIPTION OF THE DRAWINGS

The included drawings are used to provide further understanding of the embodiments of the present application, which constitute a part of the description and are used to illustrate the implementations of the present application and explain the principles of the present application together with textual description. Evidently, the drawings in the following description are merely some embodiments of the present application, and those of ordinary skill in the art may obtain other implementations according to the drawings without involving inventive effort. In the drawings:
FIG. 1 is a schematic diagram of a calibration apparatus for a drive gas circuit according to an embodiment of the present application;
FIG. 2 is a schematic diagram of a drive gas circuit and a calibration apparatus according to an embodiment of the present application;
FIG. 3 is a schematic diagram illustrating calibration of a drive gas pressure sensor in a drive gas circuit by a calibration apparatus according to an embodiment of the present application;
FIG. 4 is a schematic diagram illustrating calibration of a flow control valve in a drive gas circuit according to an embodiment of the present application;
FIG. 5 is a schematic diagram illustrating calibration of a flow restrictor in a drive gas circuit according to an embodiment of the present application;
FIG. 6 is a schematic diagram of a calibration method for a driving circuit according to an embodiment of the present application; and
FIG. 7 is a schematic diagram of a medical device according to an embodiment of the present application.

### DETAILED DESCRIPTION

The aforementioned and other features of the embodiments of the present application will become apparent from the following description with reference to the drawings. In the description and drawings, specific implementations of the present application are disclosed in detail, and part of the implementations in which the principles of the embodiments of the present application may be employed are indicated. It should be understood that the present application is not limited to the described implementations. On the contrary, the embodiments of the present application include all modifications, variations, and equivalents which fall within the scope of the appended claims.

In the embodiments of the present application, the terms "first" and "second" etc., are used to distinguish different elements, but do not represent a spatial arrangement or temporal order, etc., of these elements, and these elements should not be limited by these terms. The term "and/or" includes any and all combinations of one or more associated listed terms. The terms "comprise", "include", "have", etc., refer to the presence of described features, elements, components, or assemblies, but do not exclude the presence or addition of one or more other features, elements, components, or assemblies.

In the embodiments of the present application, the singular forms "a" and "the" include the plural forms, and should be broadly construed as "a type of" or "a class of' rather than being limited to the meaning of "one". Furthermore, the term "the" should be construed as including both the singular and plural forms, unless otherwise specified in the context. In addition, the term "according to" should be construed as "at least in part according to...", and the term "based on" should be construed as "at least in part based on...", unless otherwise specified in the context.

The features described and/or illustrated for one implementation may be used in one or more other implementations in the same or similar way, be combined with features in other implementations, or replace features in other implementations.

Embodiments of the present application provide a calibration apparatus for a drive gas circuit.

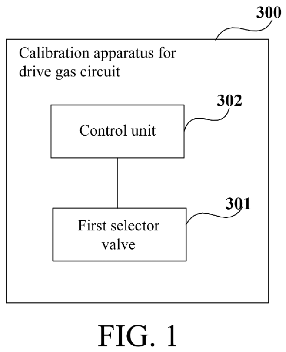

FIG. 1 is a schematic diagram of a calibration apparatus for a drive gas circuit according to an embodiment of the present application. As shown in FIG. 1, the calibration apparatus 300 for the drive gas circuit includes:
a first selector valve 301, where the first selector valve switches communication between different passages; and
a control unit 302, where the control unit calibrates different components in the drive gas circuit by controlling switching of the first selector valve 301.

According to the above embodiments, the calibration apparatus for the drive gas circuit calibrates the different components in the drive gas circuit according to the switching of communication between the different passages by the first selector valve. Thus, manual preparation is not needed before calibrating the various component in the drive gas circuit, thereby simplifying the calibration process and improving the calibration efficiency and accuracy.

In the embodiments of the present application, the drive gas circuit may be assembled on a medical device for medical gas delivery. The drive gas circuit provides certain power for fresh gas by delivering drive gas to drive delivery of the fresh gas. That is, the drive gas does not directly enter the body of a patient, but serves as a power source to drive the fresh gas and deliver it to the patient for inhalation, where the fresh gas may be a mixed gas containing an anesthetic gas with a certain concentration, or a mixed gas containing oxygen with a certain concentration or other medications.

For example, the drive gas circuit may be assembled on an anesthesia machine to drive anesthetic gas with a certain concentration and deliver it to the patient. Alternatively, the drive gas circuit may also be assembled on a nebulizer to drive mixed gas with a certain medication concentration and deliver it to the patient. In addition, the drive gas circuit may also be assembled on other medical devices for medical gas delivery, such as a medical oxygen supply system and a negative pressure suction apparatus, which is not limited in the present application.

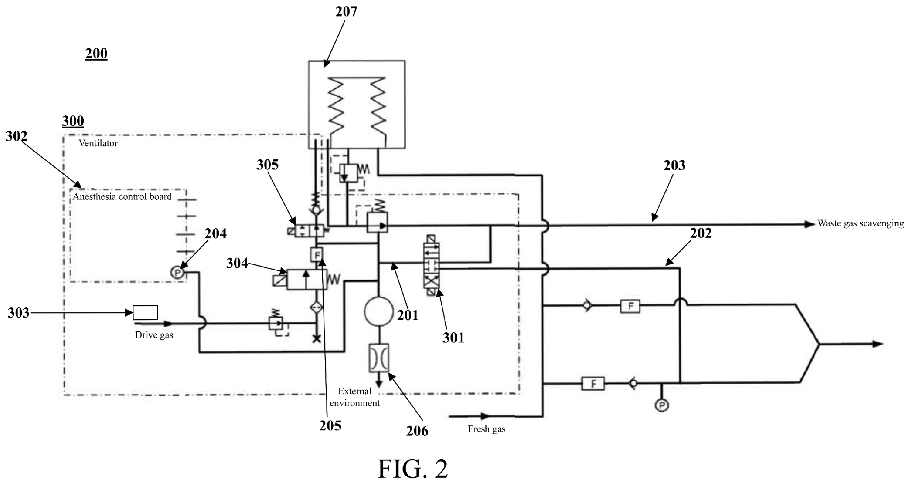

FIG. 2 is a schematic diagram of a drive gas circuit and a calibration apparatus according to an embodiment of the present application.

In some embodiments, as shown in FIG. 2, the calibration apparatus 300 includes a first selector valve 301 and a control unit 302, and further includes: an intake valve 303 that controls gas input into the passage; and a flow control valve 304, where the control unit 302 controls the flow control valve 304 to increase or decrease a flow rate of the gas in the passage.

In the embodiments of the present application, the drive gas may be compressed air, oxygen, or other mixed gases. The type and concentration of the drive gas may be set according to the specific use of the drive gas circuit. For example, when the drive gas circuit 200 is used to anesthetize the patient, the intake valve 303 inputs compressed gas into the passage to drive the anesthetic gas, and when it is necessary to provide oxygen support for the patient, the intake valve 303 inputs oxygen with a certain concentration into the passage to provide driving force.

In the embodiments of the present application, the flow rate of the drive gas in the passage is a volume of gas passing through the passage per unit time, which reflects a flow speed and amount of the gas in the passage. The unit of the flow rate of the drive gas may be ml/min or l/min, or may be set to other units, which is not limited in the present application. In addition, the flow control valve 304 controls the flow rate of the drive gas by adjusting a size of a throttle orifice inside the valve. When the throttle orifice widens, the resistance of the gas during passing decreases, and the flow increases. Conversely, when the throttle orifice narrows, the resistance of the gas during passing increases, and the flow decreases. Alternatively, the flow rate of the gas may also be directly controlled by controlling an opening degree of the valve, which is not limited in the present application. As shown in FIG. 2, the flow rate of the gas adjusted by the flow control valve 304 may be detected by a drive gas flow sensor 205.

Taking FIG. 2 as an example, the drive gas circuit 200 inputs the drive gas into the passage via the intake valve 303, and the flow control valve 304 can increase or decrease the flow rate of the drive gas input into the passage via the intake valve 303 as needed to change the driving force of the gas. The control unit 302 can control the first selector valve 301 to switch communication between the different passages. Taking FIG. 2 as an example, the first selector valve 301 can control a first passage 201 to be in communication with a second passage 202, or control the first passage 201 to be in communication with a third passage 203, or control the first passage 201 to be in communication with neither the second passage 202 nor the third passage 203. Thus, the drive gas can flow through the different passages by switching of the first selector valve 301, and the control unit 302 can calibrate the different components in the drive gas circuit 200 according to different gas flow conditions.

Hereinafter, the calibration of the different components in the drive gas circuit will be described by taking the drive gas circuit assembled on the anesthesia machine as an example.

As shown in FIG. 2, the drive gas circuit 200 is configured in a vent engine of the anesthesia machine. The vent engine mainly functions to provide respiratory support for the patient during anesthesia, maintain the respiratory function of the patient, and ensure supply of oxygen or medications and discharge of carbon dioxide for the patient. The drive gas circuit 200 provides power and gas delivery passages for operation of the vent engine, and cooperates with the vent engine to achieve synchronization of respiration of the patient and gas delivery of the vent engine. For example, during inspiration, the drive gas circuit 200 controls the drive gas to drive the fresh gas to pass through the passage and enter the airway of the patient according to preset parameters. During expiration, the drive gas circuit controls the driving force of the drive gas to ensure that exhaled gas from the patient is smoothly discharged to the outside, thereby guaranteeing a smooth and safe expiratory process.

As shown in FIG. 2, the drive gas circuit 200 includes a control unit 302. The drive gas circuit 200 delivers the drive gas via the control unit 302 to drive delivery of the fresh gas, and the control unit 302 then controls the flow rate of the drive gas via the flow control valve 304.

In some examples, as shown in FIG. 2, the control unit 302 may be an anesthesia control board (ACB). The anesthesia control board controls the drive gas flow sensor 205 to obtain the flow rate of the drive gas entering each passage via the flow control valve 304, and controls the flow control valve 304 to control the flow rate of the gas in the passage. The anesthesia control board may also control a drive gas pressure sensor 204 to obtain pressure of the gas in the drive gas circuit 200, and control pressure of the gas in the passage.

In addition, taking FIG. 2 as an example, the anesthesia control board may also control opening/closing and/or opening degrees of a first switching valve 305 (i.e., a normally open valve) and a second selector valve 301 (i.e., a reversing valve) in the drive gas circuit 200, and the anesthesia control board may also control other components in the drive gas circuit 200. Reference may be made to the related art for the specific structure of the anesthesia control board, which will not be repeated in the present application.

As shown in FIG. 2, when the drive gas circuit 200 is in an operating state, that is, when respiratory support is provided to the patient via the drive gas circuit 200, the gas generated by the drive gas circuit 200 enters a drive gas circuit passage via the passage, and the drive gas in the drive gas circuit passage delivers the fresh gas to the patient via a drive bellows, thereby providing respiratory support for the patient. Pressure in the drive gas circuit passage will change with different operating stages and can be detected by the drive gas pressure sensor 204. In addition, the control unit 302 can also detect a value of the drive gas pressure sensor 204 and adjust operating parameters of the vent engine according to a detection result.

There are operating modes inside the vent engine that correspond to different gas flow rates and pressures. For example, in some modes, the vent engine provides oxygen for the patient at a high flow rate and pressure to help the patient overcome dyspnea, and in some other modes, the vent engine maintains normal breathing of the patient at a low flow rate and pressure. However, during long-term operation of the vent engine, due to aging and wear of electronic components, the actual operating state of the vent engine deviates from the preset flow and pressure values, which is referred to as a "drift" phenomenon, and when the vent engine "drifts", the detection accuracy of the drive gas pressure sensor 204 will be affected, so in practical applications, it is necessary to calibrate the drive gas pressure sensor 204.

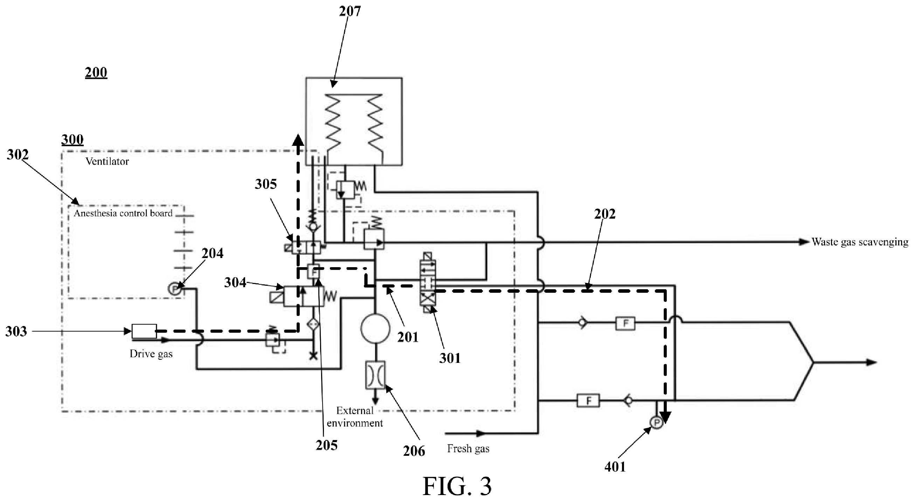

FIG. 3 is a schematic diagram illustrating calibration of a drive gas pressure sensor in a drive gas circuit by a calibration apparatus according to an embodiment of the present application.

In some embodiments, as shown in FIG. 3, when the first selector valve 301 enables communication between a first passage 201 and a second passage 202, the calibration apparatus 300 calibrates the drive gas pressure sensor 204, where the second passage 202 is in communication with a calibrated airway pressure sensor 401.

In the above embodiments, as shown in FIG. 3, when the calibration apparatus 300 calibrates the drive gas pressure sensor 204, a flow direction of the gas input into the passage via the intake valve 303 is shown by the thick dashed line in FIG. 3, the first selector valve 301 is switched to enable communication between the first passage 201 and the second passage 202, the first passage 201 is in communication with the drive gas pressure sensor 204 and a bellows cover 207 at a drive gas end, and the second passage 202 is in communication with the calibrated airway pressure sensor 401. That is, when the first passage 201 is in communication with the second passage 202 via the first selector valve 301, the airway pressure sensor 401 can be in communication with the drive gas pressure sensor 204. In other words, the pressure detected by the airway pressure sensor 401 is the same as that in the drive gas pressure sensor 204. In this case, the control unit 302 can calibrate the drive gas pressure sensor 204 according to the airway pressure sensor 401.

The calibrated airway pressure sensor 401 is an airway pressure sensor used when the drive gas circuit 200 operates normally, that is, when the drive gas circuit 200 operates normally, the airway pressure sensor 401 can read pressure in the airway of the patient.

Thus, during calibration of the drive gas pressure sensor 204, the drive gas pressure sensor 204 can be calibrated using the existing sensor in the drive gas circuit 200 by changing the connection between the passages, without additional configuration of a pressure sensor for calibration, which can simplify the structure of the drive gas circuit 200 and improve the calibration efficiency. In addition, for the calibration of the airway pressure sensor 401, reference may be made to the calibration of the airway pressure sensor capable of reading the pressure in the airway of the patient in the related art, which will not be repeated in the present application.

In some embodiments, the control unit 302 controls the flow control valve 304 to increase the flow rate of the gas in the passage from low to high, and when the airway pressure sensor 401 detects that the pressure in the passage reaches a first threshold, the control unit 302 calibrates the drive gas pressure sensor 204 according to a counter in the flow control valve 304.

The control unit 302 controls a count value of the flow control valve 304 to increase the flow rate of the gas in the passage from low to high, where the count value of the flow control valve 304 is a digital indicator for quantifying and controlling the valve opening degree of the flow control valve 304 or the flow rate of the gas in the passage, and is generally positively correlated with the valve opening degree of the flow control valve 304 and the flow rate of the gas in the passage.

For example, as shown in FIG. 3, when the count value of the flow control valve 304 increases, the valve opening degree of the flow control valve 304 increases, the flow rate of the gas in the passage shown by the thick dashed line increases, and the pressure of the gas in the passage also increases at this time; and when the airway pressure sensor 401 detects that the pressure of the gas in the passage reaches 75 cmH₂O, the control unit 302 stops increasing the count value of the flow control valve 304, and records the count value of the flow control valve 304 at this time, that is, the count value of the flow control valve 304 at this time indicates the valve opening degree of the flow control valve 304 and the flow rate of the gas in the passage at the specific pressure of 75 cmH₂O.

In the embodiments of the present application, the drive gas pressure sensor 204 is calibrated according to a pressure range detected by the drive gas pressure sensor 204 within a fixed pressure range. For example, the control unit 302 subtracts a count value of the drive gas pressure sensor 204 at zero offset from a count value of the drive gas pressure sensor 204 at a pressure of 75 cmH₂O to obtain a pressure span value of the drive gas pressure sensor 204 in a range of 0-75 cmH₂O. If the span value is within a preset range, the control unit 302 saves related data, and the calibration of the drive gas pressure sensor 204 is completed.

For the count value of the drive gas pressure sensor 204, a pressure signal measured by the drive gas pressure sensor 204 is converted into an electrical signal. The electrical signal is subjected to analog-to-digital conversion (ADC) to obtain a digital count value (which may also be referred to as an ADC value). This digital count value is the count value of the drive gas pressure sensor 204, that is, the count value of the drive gas pressure sensor 204 at 75 cmH₂O is an ADC value of the drive gas pressure sensor 204 when the pressure of the gas in the passage reaches 75 cmH₂O. The count value of the drive gas pressure sensor 204 at the zero offset is an ADC value of the drive gas pressure sensor 204 when the drive gas circuit 200 is in a zero pressure state, that is, when no pressure is applied to the drive gas pressure sensor 204. Generally, the calculation of the count value of the drive gas pressure sensor 204 at the zero offset may be automatically completed by a control system of the anesthesia machine, for example, after the calibration of the airway pressure sensor 401 is completed, the control system of the anesthesia machine automatically records the count value of the drive gas pressure sensor 204 when the pressure is zero at this time.

In the above embodiments, due to an error of the drive gas circuit 200 or an inherent error of the system itself, even if the pressure in the passage is 0, the ADC value of the drive gas pressure sensor 204 is not necessarily 0, and the count value of the drive gas pressure sensor 204 at the zero offset is used, which can eliminate the influence of the inherent error on a calibration result and improve the calibration accuracy.

According to the above embodiments, 75 cmH₂O is a measured value of the calibrated airway pressure sensor 401. Taking 75 cmH₂O as a reference value, the span value of the drive gas pressure sensor 204 represents a span value of pressure measured by the drive gas pressure sensor 204 at an actual pressure of 0-75 cmH₂O, and this span value is compared with a preset threshold. If the span value is too large, it means that the drive gas pressure sensor 204 is too sensitive to pressure changes, which easily leads to unstable measurement results and poor accuracy. If the span value is too small, it means that the drive gas pressure sensor 204 is not sensitive enough to pressure changes and has difficulty in capturing minor pressure changes, thereby affecting timely adjustment of the parameters of the drive gas circuit 200. Therefore, the drive gas pressure sensor 204 needs to be able to accurately sense the pressure changes and maintain the stability of measured data, and only when the span value is within the preset range, the drive gas pressure sensor 204 can be calibrated and perform subsequent operations normally, where the preset span range may be set differently according to different requirements, for example, if it is desired to capture the slight change of the pressure in the drive gas pressure sensor 204, a large span range may be set, and if it is desired to make the pressure in the drive gas pressure sensor 204 more stable, a small span range may be set. In addition, in the above embodiments, the reference value is set to 75 cmH₂O, and the span value of the drive gas pressure sensor 204 at 0-75 cmH₂O is detected. The present application is not limited thereto. The reference value may also be set to other values, or the unit of the reference value is set to other units than cmH₂O.

The calibration of the drive gas pressure sensor in the drive gas circuit has been described above. In the above example, since the drive gas pressure sensor 204 is in communication with the first passage 201, the first passage 201 may be in communication with the second passage 202 directly via the first selector valve 301 to calibrate the drive gas pressure sensor 204. However, in some other examples, when other components in the drive gas circuit are calibrated, it may not be necessary for the components to be in communication with the first passage. In this case, other valves need to be further used to control the first passage in the drive gas circuit.

In some embodiments, as shown in FIG. 2, the calibration apparatus 300 further includes a first switching valve 305 located in the first passage 201, where the control unit 302 controls the first switching valve 305 to switch between an open state and a closed state.

Thus, the first switching valve 305 can be opened or closed to control whether some components in the drive gas circuit 200 are in communication with the first passage 201. Taking FIG. 3 as an example, when the first switching valve 305 is in an open state, the bellows cover 207 at the drive gas end is in communication with the first passage 201; and when the first switching valve 305 is in a closed state, the bellows cover 207 at the drive gas end fails to be in communication with the first passage 201.

It should be noted that the first switching valve 305 is in the closed state only when some components in the drive gas circuit 200 are calibrated, that is, when the drive gas circuit 200 is operating normally, the first switching valve 305 is in the open state by default, which facilitates flow of the gas in the first passage, or when the drive gas pressure sensor 204 is calibrated or other components that need to be in communication with the first passage 201 during calibration are calibrated, the first switching valve 305 is also in the open state by default. Thus, more other components in the drive gas circuit 200 can be calibrated by opening and closing the first switching valve 305.

In the embodiments of the present application, as shown in FIG. 2, the control unit 302 in the drive gas circuit 200 increases or decreases the flow rate of the gas in the passage by controlling the count value of the flow control valve 304 to ensure that the flow rate of the gas entering the flow control valve 304 always meets the inspiratory demand of the patient. The flow control valve 304 is usually opened during inspiration by the patient and closed during expiration or delivers a small flow of gas to provide positive end expiratory pressure (PEEP). The flow control valve 304 accurately controls a flow rate and pressure of the fresh gas driven to enter the airway of the patient during inspiration or exhalation according to the preset parameters by controlling the opening and closing degree of the valve to meet normal gas supply or other requirements, that is, when the drive gas circuit 200 is in the operating state, the flow control valve 304 regulates the flow rate of the drive gas in the passage, and further distributes the drive gas content according to the real-time demand during each inspiration by the patient to ensure that the patient obtains a stable and accurate gas supply during inspiration. Therefore, the flow control valve 304 requires high accuracy in measuring the flow rate of the drive gas during operation, making it necessary to calibrate the flow control valve 304.

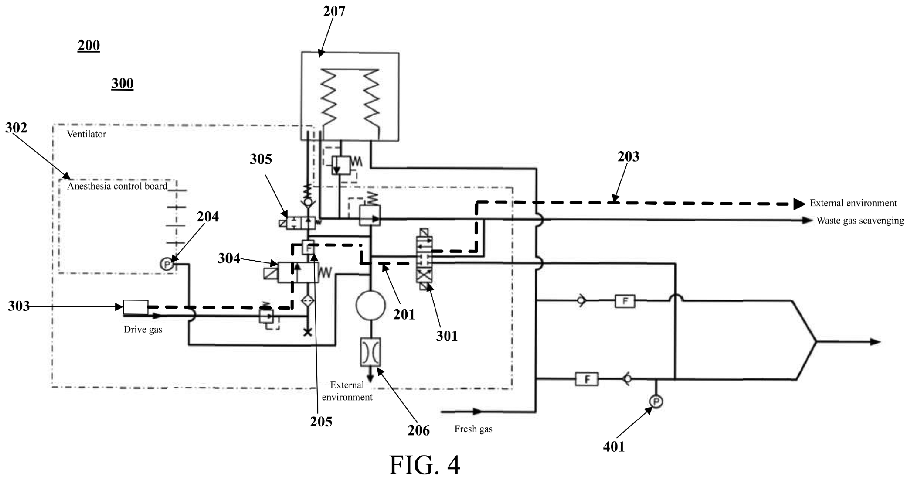

FIG. 4 is a schematic diagram illustrating calibration of a flow control valve in a drive gas circuit according to an embodiment of the present application.

In some embodiments, when the first selector valve 301 enables communication between the first passage 201 and a third passage 203, and the first switching valve 305 is closed, the calibration apparatus 300 calibrates the flow control valve 304, where the third passage 203 is in communication with the outside.

In the above embodiments, as shown in FIG. 4, when the calibration apparatus 300 calibrates the flow control valve 304, the flow direction of the gas input into the passage via the intake valve 303 is shown by the thick dashed line shown in FIG. 4, and it can be seen that the first selector valve 301 is switched to enable communication between the first passage 201 and the third passage 203, and the first switching valve 305 is closed. Therefore, the gas can only flow from the first passage 201 to the third passage 203, and does not flow to the bellows cover 207 at the drive gas end or other components connected to the first passage 201. The third passage 203 is in communication with the external environment, that is, the gas input into the passage at this time can flow to the external environment via the flow control valve 304, the first passage 201 and the third passage 203 respectively, such that the flow control valve 304 can be calibrated.

When the flow control valve 304 is calibrated, the third passage 203 is in communication with the external environment, and when the drive gas circuit 200 is in the operating state, as shown in FIG. 4, the third passage 203 can be in communication with the external environment to perform waste gas scavenging. Thus, the passage used by the drive gas circuit 200 in the operating state is used to be in communication with the external environment, thereby avoiding additional configuration of a separate passage for calibration, simplifying the gas circuit structure, and improving the calibration efficiency.

In some embodiments, the control unit 302 controls count values of the flow control valve 304 to be different values, and controls a drive gas flow sensor 205 to record an air flow value corresponding to each count value, thereby establishing a flow lookup table of a correspondence between the count values and the corresponding air flow values.

The control unit 302 controls the opening and closing degree of the flow control valve 304 by controlling the count values of the flow control valve 304 to be the different values, and obtains actual air flow values that change according to detection results of the drive gas flow sensor 205, thereby establishing the flow lookup table of the correspondence between the count values of the flow control valve 304 and the corresponding air flow values, which means that an accurate correspondence between the count values of the flow control valve 304 and the actual flow rates is established. That is, when the drive gas circuit 200 is in the operating state, the control unit 302 can determine the actual gas flow values corresponding to the count values according to the flow lookup table, thereby accurately controlling the flow control valve 304. Thus, the flow control valve 304 is calibrated.

In the above embodiments, the flow control valve 304 can be calibrated via the above calibration process. The present application is not limited thereto. When other control valves, such as other inspiratory valves, need to be calibrated, the above operation may also be adopted.

The calibration of the flow control valve in the drive gas circuit has been described above, and the calibration of a flow restrictor is described below.

In the embodiments of the present application, as shown in FIG. 2, the drive gas circuit 200 further includes a flow restrictor 206 (which may also be referred to as a flow limiter or a throttle), where the flow restrictor 206 is in communication with the passage in the drive gas circuit 200, and functions to regulate and control the pressure in the passage in the drive gas circuit 200, so as to ensure safe and stable operation of the drive gas circuit. When the gas flows in the passage, the flow restrictor 206 can impede flow of the gas, thereby regulating the flow rate of the gas or the pressure in the drive gas pressure sensor 204.

For example, the flow restrictor 206 allows the gas in the drive gas circuit 200 to flow out in a controllable manner. The greater the flow rate of the gas passing through the flow restrictor 206, the higher the pressure in the drive gas circuit 200. By controlling a small flow of gas passing through the flow restrictor 206, pilot pressure in an expiratory valve (not shown in the figure) may be controlled according to a mapping relationship between the flow rate of the gas in the flow restrictor 206 and the pressure in the drive gas circuit 200. Thus, the flow restrictor 206 can ensure that the entire drive gas circuit 200 is within a normal pressure range. When the flow restrictor 206 has a fault (for example, the flow restrictor 206 is blocked), the actual pressure detected in the drive gas pressure sensor 204 does not match the expected pressure, which may damage the passage or the drive gas pressure sensor 204. Therefore, it is necessary to calibrate the flow restrictor 206.

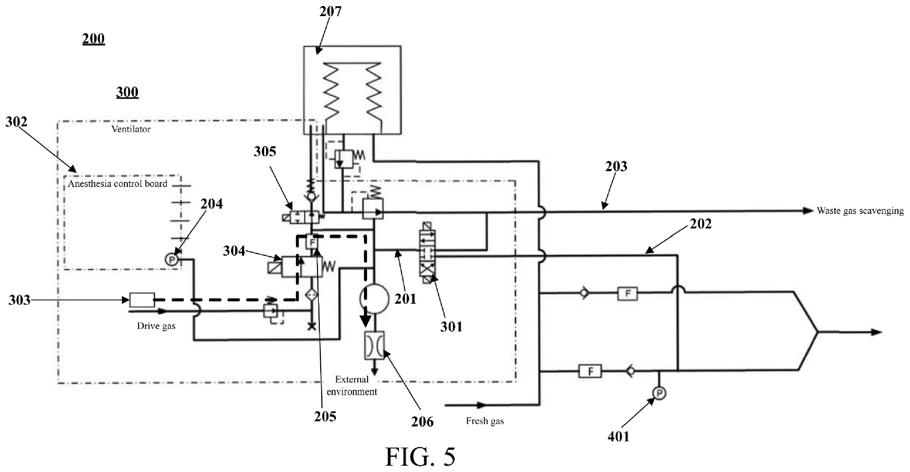

FIG. 5 is a schematic diagram illustrating calibration of a flow restrictor in a drive gas circuit according to an embodiment of the present application.

In some embodiments, when the first selector valve 301 and the first switching valve 305 are closed, the calibration apparatus 300 calibrates the flow restrictor 206, where the first passage 201 is in communication with the flow restrictor 206.

In the above embodiments, as shown in FIG. 5, when the calibration apparatus 300 calibrates the flow restrictor 206, the flow direction of the gas input into the passage via the intake valve 303 is shown by the thick dashed line in FIG. 5, and it can be seen that the first selector valve 301 is switched to the closed state, that is, the first passage 201 is not in communication with either the second passage 202 or the third passage 203, and the first switching valve 305 is closed. Thus, the gas flowing in via the intake valve 303 can only flow to the flow restrictor 206, and the control unit 302 can calibrate the flow restrictor 206.

In some embodiments, as shown in FIG. 5, the control unit 302 controls counting of the flow control valve 304 to increase a flow rate of gas in the gas circuit, and the drive gas flow sensor 205 detects the flow rate of the drive gas, thereby establishing a calibration table of a correspondence between a flow value of the drive gas and the pressure in the drive gas pressure sensor 204 to calibrate the flow restrictor 206 according to the calibration table.

In the above embodiments, the control unit 302 controls the count of the flow control valve 304 to increase, such that the flow rate of the drive gas in the gas circuit increases. When the flow rate of the drive gas in the gas circuit increases, the pressure detected by the drive gas pressure sensor 204 also increases, thereby obtaining a calibration table reflecting the correspondence between the flow rate of the gas in the gas circuit and the pressure in the drive gas pressure sensor 204. This table reflects the performance of the flow restrictor 206, and indicates whether the flow restrictor 206 can maintain the pressure in the drive gas pressure sensor 204 within a stable range under different gas flow rates. Reference may be made to the related art for calibration of the flow restrictor 206 according to the calibration table, which is not limited in the present application.

In some embodiments, the calibration apparatus 300 automatically starts a next calibration process after completing calibration of the drive gas pressure sensor 204, the flow control valve 304, or the flow restrictor 206. The calibration of the drive gas pressure sensor, the flow control valve, and the flow restrictor may be performed individually or in combination of two or more. The present application is not limited thereto.

In some embodiments, the calibration apparatus 300 starts the calibration of the flow restrictor 206 after completing the calibration of the drive gas pressure sensor 204. Since the calibration apparatus 300 needs to establish the calibration table of the correspondence between the pressure in the flow restrictor 206 and the flow rate according to the value of the drive gas pressure sensor 204 when calibrating the flow restrictor 206, it is necessary to calibrate the drive gas circuit pressure sensor 204 before calibrating the flow restrictor 206.

In the above embodiments, for the calibration of the different components in the drive gas circuit 200, after the calibration of one component is completed, the calibration of the next component may be automatically started. For example, after the calibration of the drive gas pressure sensor 204 is completed, the calibration of the flow control valve 304 is automatically started; after the calibration of the flow control valve 304 is completed, the calibration of the flow restrictor 206 is automatically started; and the calibration of other components is sequentially completed until the calibration of the entire drive circuit 200 is completed. The calibration order of the various components may generally be interchanged. However, for calibration of the flow restrictor 206, since the drive gas pressure sensor 204 is required to measure the gas pressure during calibration, the drive gas pressure sensor 204 may be calibrated first before calibrating the flow restrictor 206.

As described above, taking the anesthesia machine as an example, calibration of the drive gas pressure sensor 204, the flow control valve 304, and the flow restrictor 206 in the drive gas circuit of the anesthesia machine has been described. However, the present application is not limited thereto. Other components in the drive gas circuit may be calibrated by switching the first selector valve 301 and opening or closing the first switching valve 305. Other valves may be further provided to switch the passage and calibrate other components as needed. Alternatively, the drive gas pressure sensor, the flow control valve, and the flow restrictor, or other components in the drive gas circuit of other medical devices may also be calibrated, which is not limited in the present application.

It is worth noting that only the components or modules related to the present application have been described above, but the present application is not limited thereto. The calibration apparatus 300 for the drive gas circuit 200 may further include other components or modules, and reference may be made to the related art for details of these components or modules.

For the sake of simplicity, FIG. 2 to FIG. 5 only exemplarily illustrate connection relationships or signal directions between various components or modules, but it should be clear to those skilled in the art that various related technologies such as bus connection can be used. The various components or modules described above can be implemented by means of hardware such as a processor or a memory, etc. The embodiments of the present application are not limited thereto.

According to the above embodiment, the calibration apparatus of the drive gas circuit can calibrate different components in the drive gas circuit by switching the communication between different passages according to the first selector valve, such that there is no need to manually perform a preparation process before calibrating each component of the drive gas circuit, which simplifies the calibration process and improves the calibration efficiency and calibration accuracy.

The embodiments of the present application further provide a calibration method for a drive gas circuit. The same content as that of the foregoing embodiments will not be repeated.

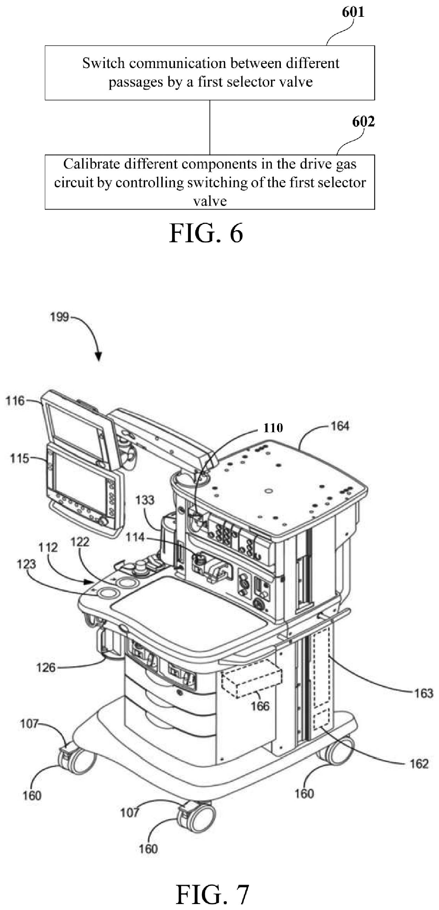

FIG. 6 is a schematic diagram of a calibration method for a drive gas circuit according to an embodiment of the present application. As shown in FIG. 6, the calibration method 600 for the drive gas circuit includes:
601: switching communication between different passages by a first selector valve; and
602: calibrating different components in the drive gas circuit by controlling switching of the first selector valve.

In some embodiments, the drive gas circuit controls gas input into the passage by an intake valve; and the drive gas circuit controls a flow control valve to increase or decrease a flow rate of the gas in the passage.

In some embodiments, when the first selector valve enables communication between a first passage and a second passage, a drive gas pressure sensor is calibrated, where the second passage is in communication with a calibrated airway pressure sensor.

In some embodiments, the flow control valve is controlled to increase the flow rate of the gas in the passage from low to high, and when the airway pressure sensor detects that pressure in the passage reaches a first threshold, the drive gas pressure sensor is calibrated.

In some embodiments, a first switching valve located in the first passage is controlled to switch between an open state and a closed state.

In some embodiments, when the first selector valve enables communication between the first passage and a third passage, and the first switching valve is closed, the flow control valve is calibrated, where the third passage is in communication with the outside.

In some embodiments, count values of the flow control valve are controlled to be different values, and a drive gas flow sensor is controlled to record an air flow value corresponding to each of the count values, thereby establishing a flow lookup table of a correspondence between the count values and the corresponding air flow values to complete calibration of the flow control valve.

In some embodiments, when the first selector valve and the first switching valve are closed, a flow restrictor is calibrated, where the first passage is in communication with the flow restrictor.

In some embodiments, counting of the flow control valve is controlled to increase a flow rate of gas in the gas circuit, and a calibration table of a correspondence between a gas flow value and pressure in the drive gas pressure sensor is established to calibrate the flow restrictor according to the calibration table.

In some embodiments, a next calibration process is automatically started after completing calibration of the drive gas pressure sensor, the flow control valve, or the flow restrictor.

In some embodiments, the calibration of the flow restrictor is started after completing the calibration of the drive gas pressure sensor.

It is worth noting that FIG. 6 merely schematically illustrates an embodiment of the present application, but the present application is not limited thereto. For example, the order of execution between operations may be appropriately adjusted. In addition, some other operations may be added or some operations may be omitted. Those skilled in the art may make appropriate variations according to the above content, rather than being limited to what is set forth in FIG. 6.

The embodiments of the present application further provide a medical device, including a driving circuit, where the medical device further includes the calibration apparatus for the driving circuit according to the foregoing embodiments.

FIG. 7 is a schematic diagram of a medical device according to an embodiment of the present application. As shown in FIG. 7, the medical device 199 may be an anesthesia machine or an anesthesia system. The anesthesia machine 199 includes a frame 164 supported by casters 160, where the movement of the casters may be controlled (e.g., stopped) by one or more locks 107. The anesthesia machine 199 further includes a breathing gas module 110, a ventilator 112, a vaporizer 114, an anesthesia display apparatus 115, and a patient monitoring display apparatus 116.

In some embodiments, one or more of conduit connectors and/or gas cylinders may be a mixer gas source configured to provide gas to a gas mixer 163 of the anesthesia machine (schematically shown in FIG. 7). The gas entering the anesthesia machine may be mixed with a vaporized anesthetic agent in the vaporizer and may be supplied to the patient via the gas mixer.

The ventilator 112 may include an expiratory check valve 122, an inspiratory check valve 123, an absorbent canister 126, and a bellows-less pressure chamber 133. When a patient breathing circuit is coupled to the ventilator, breathing gas (e.g., fresh gas, oxygen, and/or nitrous oxide mixed with the vaporized anesthetic agent) exits the machine from an inspiratory port (which may be co-located with the inspiratory check valve 123) and flows to the patient. During standard ventilation, exhaled gas from the patient may re-enter the anesthesia machine via an expiratory port (which may be co-located with the expiratory check valve 122), where carbon dioxide may be removed from the exhaled gas via the absorbent canister 126. However, during some clinician applications (such as intubation with high-flow nasal cannulas), the expiratory valve of the anesthesia machine may be maintained in a closed position, and the patient may exhale inhaled gas to the atmosphere.

During operation of the vaporizer 114, an operator (e.g., an anesthesiologist, a clinician, etc.) may adjust the amount of the vaporized anesthetic agent supplied to the patient by regulating the flow rate of gas from the gas source (e.g., one or more of gas conduits) of the gas mixer to the vaporizer. The ventilator 112 may optionally be coupled to the breathing circuit (not shown) that includes a plurality of tubes (e.g., gas passages). The breathing circuit may be coupled between the airway of the patient (e.g., via a breathing mask positioned to enclose the mouth and/or nose of the patient) and the inspiratory port. Gas (e.g., oxygen or a mixture of the oxygen and the vaporized anesthetic agent from the vaporizer 114) may flow through the breathing circuit from the port and enter the airway of the patient, where the gas is absorbed by the lung of the patient. By adjusting the concentration of the vaporized anesthetic agent in the gas as described above, the operator may adjust the amount of anesthesia to the patient. The ventilator 112 may be an electronic ventilator controlled via an electronic controller 162, and the operation of the ventilator may be adjusted according to user-selected parameters. For example, in a state where a user (e.g., a clinician) selects a high-flow mode of the anesthesia machine, the controller 162 may control the ventilator 112 to flow ventilator-driven gas from one or more ventilator-driven gas sources through the bellows-less pressure chamber to the inspiratory port. However, in a state where the high-flow mode is not selected, the controller 162 may control the ventilator 112 to provide ventilator-driven gas to the bellows-less pressure chamber, thereby driving the mixer gas flowing from the gas mixer to the inspiratory port, and preventing the ventilator-driven gas from flowing to the inspiratory port.

In a state where the breathing circuit is coupled to the airway, the anesthetic agent and/or the fresh gas may flow into the airway (e.g., be inhaled by the patient) via the inspiratory check valve 123. For example, the inspiratory check valve 123 may automatically open in response to inhalation by the patient (e.g., without input or adjustment by the operator) and may automatically close in response to exhalation by the patient. Similarly, the expiratory check valve 122 may automatically open in response to exhalation by the patient and may automatically close in response to inhalation by the patient.

In some examples, the operator may alternatively and/or additionally control one or more operating parameters of the anesthesia machine 199 via the electronic controller 162 of the anesthesia machine 199 (schematically shown in FIG. 1). The controller 162 includes a processor operatively connected to a memory. The memory may be a non-transitory computer-readable medium and may be configured to store computer-executable codes (e.g., instructions) to be processed by the processor in order to perform one or more routines as described herein. The memory may be further configured to store data received by the processor. The controller 162 may be communicatively (e.g., wired or wirelessly) coupled to one or more external or remote computing devices, such as a hospital computing system, and may be configured to send and receive various information, such as electronic medical record information and procedure information. In some embodiments, the controller 162 may control operation of a flush module 166 to perform one or more cleaning cycles, disinfection cycles, flushing cycles, or the like for a gas path of the anesthesia machine 199.

The above description is made only by taking the medical device as an anesthesia machine. However, the present application is not limited thereto. The medical device may also be other devices, such as a nebulizer or other fluid delivery devices.

The above embodiments merely provide illustrative descriptions of the embodiments of the present application. However, the present application is not limited thereto, and suitable variations may be made on the basis of the above embodiments. For example, each of the above embodiments may be used independently, or one or more of the above embodiments may be combined.

The embodiments of the present application further provide a computer-readable program, where the program, when executed in a medical imaging system, causes a computer to perform, in the medical imaging system, the calibration method for the drive gas circuit according to the foregoing embodiments.

The embodiments of the present application further provide a storage medium storing a computer-readable program, where the computer-readable program causes a computer to perform, in a medical imaging system, the calibration method for the drive gas circuit according to the foregoing embodiments.

According to the above embodiment, the calibration apparatus of the drive gas circuit can calibrate different components in the drive gas circuit by switching the communication between different passages according to the first selector valve, such that there is no need to manually perform a preparation process before calibrating each component of the drive gas circuit, which simplifies the calibration process and improves the calibration efficiency and calibration accuracy.

The above apparatus and method of the present application can be implemented by hardware, or can be implemented by hardware in combination with software. The present application relates to such a computer-readable program that when executed by a logic component, the program causes the logic component to implement the foregoing apparatus or a constituent component, or causes the logic component to implement various methods or steps as described above. The present application further relates to a storage medium for storing the above program, such as a hard disk, a disk, an optical disk, a DVD, a flash memory, etc.

The method/apparatus described in view of the embodiments of the present application may be directly embodied as hardware, a software module executed by a processor, or a combination of the two. For example, one or more of the functional block diagrams and/or one or more combinations of the functional block diagrams shown in the drawings may correspond to either respective software modules or respective hardware modules of a computer program flow. The foregoing software modules may respectively correspond to the steps shown in the figures. The foregoing hardware modules can be implemented, for example, by firming the software modules using a field-programmable gate array (FPGA).

The software modules may be located in a RAM, a flash memory, a ROM, an EPROM, an EEPROM, a register, a hard disk, a portable storage disk, a CD-ROM, or any other form of storage medium known in the art. The storage medium may be coupled to a processor, such that the processor can read information from the storage medium and can write information into the storage medium. Alternatively, the storage medium may be a constituent component of the processor. The processor and the storage medium may be located in an ASIC. The software module may be stored in a memory of a mobile terminal, and may also be stored in a memory card that can be inserted into a mobile terminal. For example, if a device (such as a mobile terminal) uses a large-capacity MEGA-SIM card or a large-capacity flash memory apparatus, the software modules can be stored in the MEGA-SIM card or the large-capacity flash memory apparatus.

One or more of the functional blocks and/or one or more combinations of the functional blocks shown in the accompanying drawings may be implemented as a general-purpose processor, a digital signal processor (DSP), an application-specific integrated circuit (ASIC), a field-programmable gate array (FPGA) or other programmable logic devices, discrete gate or transistor logic devices, a discrete hardware assembly, or any appropriate combination thereof for executing the functions described in the present application. The one or more functional blocks and/or the one or more combinations of the functional blocks shown in the accompanying drawings may also be implemented as a combination of computing devices, such as a combination of a DSP and a microprocessor, multiple microprocessors, one or more microprocessors in communication combination with a DSP, or any other such configuration.

The present application is described above with reference to specific implementations. However, it should be clear to those skilled in the art that the foregoing description is merely illustrative and is not intended to limit the scope of protection of the present application. Various variations and modifications may be made by those skilled in the art according to the principle of the present application, and said variations and modifications also fall within the scope of the present application.

## Claims

1. A calibration apparatus for a drive gas circuit, comprising:
a first selector valve, the first selector valve switching communication between different passages; and
a control unit, the control unit calibrating different components in the drive gas circuit by controlling switching of the first selector valve.

2. The calibration apparatus according to claim 1, further comprising:
an intake valve, the intake valve controlling gas input into the passage; and
a flow control valve, the control unit controlling the flow control valve to increase or decrease a flow rate of the gas in the passage.

3. The calibration apparatus according to claim 2, **characterized in that**,
when the first selector valve enables communication between a first passage and a second passage, the calibration apparatus calibrates a drive gas pressure sensor, wherein the second passage is in communication with a calibrated airway pressure sensor.

4. The calibration apparatus according to claim 3, **characterized in that**,
the control unit controls the flow control valve to increase the flow rate of the gas in the passage from low to high, and when the airway pressure sensor detects that pressure in the passage reaches a first threshold, the control unit calibrates the drive gas pressure sensor.

5. The calibration apparatus according to claim 2, further comprising:
a first switching valve, the first switching valve being located in a first passage, and the control unit controlling the first switching valve to switch between an open state and a closed state.

6. The calibration apparatus according to claim 5, **characterized in that**,
when the first selector valve enables communication between the first passage and a third passage, and the first switching valve is closed, the calibration apparatus calibrates the flow control valve, wherein the third passage is in communication with the outside.

7. The calibration apparatus according to claim 6, wherein
the control unit controls count values of the flow control valve to be different values, and controls a drive gas flow sensor to record an air flow value corresponding to each of the count values, thereby establishing a flow lookup table of a correspondence between the count values and the corresponding air flow values to complete calibration of the flow control valve.

8. The calibration apparatus according to claim 5, **characterized in that**,
when the first selector valve and the first switching valve are closed, the calibration apparatus calibrates a flow restrictor, wherein the first passage is in communication with the flow restrictor.

9. The calibration apparatus according to claim 8, **characterized in that**,
the control unit controls counting of the flow control valve to increase a flow rate of gas in the gas circuit, and establishes a calibration table of a correspondence between a gas flow value and pressure in a drive gas pressure sensor to calibrate the flow restrictor according to the calibration table.

10. The calibration apparatus according to any one of claims 1 to 9, **characterized in that**,
the calibration apparatus automatically starts a next calibration process after completing calibration of the drive gas pressure sensor, the flow control valve, or the flow restrictor,
wherein the calibration apparatus starts the calibration of the flow restrictor after completing the calibration of the drive gas pressure sensor.

11. A calibration method for a drive gas circuit, comprising:
switching communication between different passages by a first selector valve; and
calibrating different components in the drive gas circuit by controlling switching of the first selector valve.

12. A medical device, comprising a drive gas circuit, **characterized in that**, the medical device further comprises the calibration apparatus for the drive gas circuit according to any one of claims 1 to 10.
